(19) Europäisches Patentamt
European Patent Office
Office européen des brevets

(11) **EP 3 496 850 B1**

(12) # EUROPEAN PATENT SPECIFICATION

(45) Date of publication and mention of the grant of the patent:
**06.01.2021 Bulletin 2021/01**

(21) Application number: **17727556.7**

(22) Date of filing: **26.05.2017**

(51) Int Cl.:
***B01J 23/26*** *(2006.01)* ***B01J 23/62*** *(2006.01)*
***B01J 35/00*** *(2006.01)* ***B01J 37/02*** *(2006.01)*
***B01J 38/12*** *(2006.01)* ***B01J 38/10*** *(2006.01)*
***B01J 23/96*** *(2006.01)* ***C07C 5/00*** *(2006.01)*

(86) International application number:
**PCT/EP2017/062779**

(87) International publication number:
**WO 2018/036672 (01.03.2018 Gazette 2018/09)**

(54) **CATALYTICALLY ACTIVE COMPOSITIONS OF MATTER**
KATALYTISCH AKTIVE STOFFZUSAMMENSETZUNGEN
COMPOSITIONS DE MATIÈRE CATALYTIQUEMENT ACTIVE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.08.2016 EP 16185958**

(43) Date of publication of application:
**19.06.2019 Bulletin 2019/25**


(73) Proprietor: **Friedrich-Alexander-Universität Erlangen-Nürnberg**
**91054 Erlangen (DE)**

(72) Inventors:
- **GÖRLING, Andreas**
**82061 Neuried (DE)**
- **STEINRÜCK, Hans-Peter**
**91056 Erlangen (DE)**
- **TACCARDI, Nicola**
**90766 Fürth (DE)**
- **WASSERSCHEID, Peter**
**91054 Erlangen (DE)**
- **DEBUSCHEWITZ, Jonas**
**91560 Heilsbronn (DE)**

(74) Representative: **Grimm, Siegfried**
**E. Blum & Co. AG**
**Vorderberg 11**
**8044 Zürich (CH)**


(56) References cited:
**EP-A1- 0 637 578** **US-A- 4 224 192**
**US-A- 5 219 816**

EP 3 496 850 B1


Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention pertains to the field of catalyst and catalytic reactions. Specifically, the invention provides for new catalytically active compositions of matter, to methods of manufacturing them and to the use of such compositions. The catalytically active compositions comprise an alloy which is liquid under reaction conditions and which is adsorbed on a support material.

Catalytically active compositions for commercial applications, comprising a support material and a catalytic active component, particularly Pt and Pd, that also contain gallium in non-metallic form are well known. A number of compositions comprising Pt or Pd as a catalytically active component on a support are described in the following documents, grouped A) to F). In groups A) to D) specific binary systems on a support are discussed, in group E) ternary systems on a support are discussed and in group F) singular systems on a support are discussed. In all these documents, due to the wet impregnation starting with gallium nitrate (or indium nitrate respectively), the gallium (or indium respectively) is present in oxidized form and not as a metal.

**A)** Compositions comprising non-metallic gallium and platinum on a support material:

Wan (US4822699) discloses an improved electrocatalyst comprising Pt and Ga on a support. The electrocatalyst is suited as an electrode for fuel cells. The electrocatalysts disclosed in that document comprise high amounts of Pt and low amounts of Ga. Due to the use of gallium nitrate as a precursor, no gallium, but gallium oxide is formed.

Luo et al (US2010/0236985) disclose a catalyst comprising Pt and Ga on a support. The catalyst is suited for dehydrogenation reactions. Due to the use of gallium nitrate as a precursor, no gallium, but gallium oxide is formed.

J. Sattler et al. (Chemical Reviews, 2014, 114, 10613-10653) reviews the catalytic dehydrogenation of light alkanes on metals and metal oxides. The authors discuss commercial catalysts, suitable reaction conditions, mechanisms and drawbacks of current catalysts. Industrial applied catalyst systems for the dehydrogenation of short-chain alkanes, as for example n-butane, are platinum particles on alumina supports (Olefex process) and $Cr_2O_3$ on alumina supports (Catofin process). As main drawbacks, selectivity, conversion rate and deactivation (mainly caused due to coke formation) are identified. This document incorrectly cites in table 7 (10) Iezzi et al (reference 247 = US2002/0198428). As apparent from the original publication, the catalytic system comprises 0.001wt-%Pt, 1.2 wt-%Ga2O3 on Al2O3-support. The system of palladium-gallium oxide has a melting point far above 400°C and does not contain metallic gallium.

Foster et al (US4224192) disclose a modified alumina catalyst comprising Pt and Ga on a support. The catalyst is suited for hydrocarbon conversion reactions. Due to the use of gallium nitrate as a precursor, no gallium is formed. As specifically stated in ex.2 of that document, the gallium was incorporated in the alumina.

**B)** Compositions comprising non-metallic gallium and palladium on a support material:

Han et al (US2007/0123418) disclose a catalyst comprising Pd and Ga on a support. The catalyst is suited for purifuying exhaust gases of internal combustion engines. The catalysts disclosed in that document comprise high amounts of Pd and low amounts of Ga. Due to the use of gallium nitrate as a precursor, no gallium is formed.

Zhang et al (US2010/0303713) disclose a catalyst comprising Pd and Ga on a support. The catalyst is suited for methanol steam reforming. The catalysts disclosed in that document comprise high amounts of Pd and low amounts of Ga. Due to the use of gallium nitrate as a precursor, no gallium is formed. As indicated in that document, claim 1, Pd is present as Pd oxide and Ga is present as Ga oxide.

**C)** Compositions comprising non-metallic indium and platinum on a support material:

Gajda et al (US2010/0216630) discloses a catalyst comprising In, Sn and Pt. The catalyst is suited for reforming reactions. The catalysts disclosed in that document comprise tin as a part of the alumina support. As indicated in that document, claim 1 and abstract, Indium is present in the oxidized form while Pt is present in elemental form, thereby excluding Pt being dissolved in In.

Benderly et al (EP1533029) disclose a catalyst comprising In and Pt. The catalyst is suited for converting alkanes to alkenes and their oxygenated products. The catalysts disclosed in that document comprise high amounts of Pt and low amounts of In; at least 15% Pt are disclosed in table 1. The catalysts disclosed in that document comprise Pt / In oxides on an alumina support. As explicitly indicated in that document, example 2, indium is present in the oxidized form (In2O3) thereby excluding Pt being dissolved in In.

**D)** Compositions comprising non-metallic indium and palladium on a support material:

Men et al (DE1020090458004) disclose a catalyst comprising In and Pd. The catalyst is suited for methanol

steam reforming. The catalysts disclosed in that document comprise high amounts of Pd and low amounts of In. Further, the catalysts disclosed in that document comprise indium oxide and Pd (or Pt) on an alumina support. As indicated in that document, table 1, indium is present in the oxidized form (In2O3) while Pd/Pt is present in elemental form, thereby excluding Pt being dissolved in In.

**E)** Compositions comprising non-metallic gallium and palladium + cobalt / chromium on a support material:

Sorrentino et al (US4469812) disclose a three-component catalyst with components A, B and C. The catalyst is suited for hydrocarbon reforming. The catalysts disclosed in that document comprise high amounts of Pt and low amounts of In. This catalyst was found to be inferior compared to a catalyst where components A, B and Ca are separated on individual supports and then extruded.

Luczak et al (US1880711) disclose a ternary catalyst comprising Pt, Ga and Co or Cr. The catalyst is suited for fuel cells. As specified in that document, col.2, 1.38, the catalyst must contain at least about 50 atom-% Pt to be effective and thus less than 50 atom-% Ga + Co/Cr. Such Pt rich ternary systems have melting points way above 500°C.

**F)** Compositions comprising platinum or palladium on a support material; free of gallium or indium:

S. Veldurthi et al. (Catalysis Today, 2012, 185, 88-93) discuss promotional effects of Cu on Pt / alumina and Pd / alumina catalysts during dehydrogenation. The authors show that such modification may beneficially influence selectivity and in some instances activity of the catalyst. S. Bocanegra et al. (Catalysis Today, 2009, 143, 334-340) discuss highly selective and stable bimetallic catalysts supported on different materials for n-butane dehydrogenation. The authors show that the support influences catalyst performance.

J. Sattler et al. (Physical Chemistry Chemical Physics, 2013, 12095-12103] study the deactivation of Pt/Al2O3 and Pt-Sn/Al2O3 propane dehydrogenation catalysts. The authors conclude that addition of hydrogen beneficially influences catalyst performance and decreases the formation of coke deposits.

Although suitable, there is an ongoing need for alternative, particularly improved, catalytic systems. Specifically, the above catalytic systems are considered to suffer from one or more of the following drawbacks: insufficient activity and / or selectivity and / or fast deactivation.

In consequence, there is a need for improved catalysts, resolving one or more of the shortcomings discussed in the prior art.

Thus, it is an object of the present invention to mitigate at least some of these drawbacks of the state of the art. In particular, it is an aim of the present invention to provide catalytic systems with improved activity and / or selectivity and / or less deactivation.

These objectives are achieved by the catalytically active compositions as defined in claim 1 and by the catalytic processes as defined in claim 11. Further aspects of the invention are disclosed in the specification and independent claims, preferred embodiments are disclosed in the specification and the dependent claims.

The present invention will be described in more detail below.

Unless otherwise stated, the following **definitions** shall apply in this specification:

As used herein, the term "a", "an", "the" and similar terms used in the context of the present invention (especially in the context of the claims) are to be construed to cover both the singular and plural unless otherwise indicated herein or clearly contradicted by the context.

As used herein, the terms "including", "containing" and "comprising" are used herein in their open, non-limiting sense.

The present invention will be better understood by reference to the **figures.**

**Figs. 1-3** show the results of test runs using diluted butane as starting material. Conversion, TOF, and selectivity are shown vs. time. The results obtained using the inventive catalytic material are indicated with solid symbols.

**Figs. 4-5** show the results of test runs using pure (non-diluted) butane as starting material. TOF and selectivity are shown vs. time. The results obtained using the inventive catalytic material are indicated with solid symbols.

**Fig. 6** compares the catalytic activity (TOF*) of n-butane dehydrogenation with non-diluted starting material, where solid circle and solid triangle represent commercial CrAlOx- and Pt/AlOx-dehydriation catalysts. The inventive catalytic material is represented by a solid square.

**Fig. 7** shows catalytic activity (TOF) of n-butane dehydrogenation with non-diluted starting material in a long-term experiment over 110hrs.

**Fig. 8-9** show the results of test runs using catalysts with further transition metals and pure (non-diluted) butane as starting material, where solid circles represent a rhodium containing catalyst, solid triangles represent a platinum containing catalyst and solid squares represent a chromium containing catalyst. TOF and selectivity are shown vs. time.

**Fig. 10** shows a flow scheme of the continuous flow dehydrogenation rig used for the catalytic reaction testing. "MFC" represents the mass flow controller for the starting materials, namely inert gas (identified as "Helium") and feedstock (identified as "n-Butane"). "TIC" represents temperature indicator controllers placed in the reactor and "GC" represents analytical devices for product characterization (specifically Gas Chromatograph).

**Fig. 11** shows the known Ga-Pd phase diagram. Y-axis: temperature; x-axis (bottom) atom-% Pd; x-axis (top) wt-% Pd. The dashed horizontal region shows suitable ranges for (B+C) . As can be seen from this diagram, compositions (B+C) comprising 20 atom-% Pd (corresponding to approx. 25 wt-% Pd) show a melting point of 500°C. Accordingly, only compositions comprising a high portion of gallium (not gallium oxide) are liquid under catalytic reaction conditions.

In a **first aspect,** the invention relates to a new composition of matter consisting of a support and a coating, whereby said support contains a non-metallic and porous component (A), and said coating consists of a first metallic component (B) and a second metallic component (C), characterized in that said component (C) is catalytically active and said component (C) is present in component (B) in dissolved and / or in suspended form, and characterized in that said coating (B+C) has a melting point below 200°C, and whereby components (A) (B) and (C) are further specified as in claim 1.

The compositions disclosed herein are catalytically active and may therefore also be considered as catalytic systems or catalyst compositions.

Key features of the present invention are that component (C) is present in component (B) in dissolved and / or in suspended form (thereby forming an alloy of (B+C)), and that (B+C) has a melting point below 200°C. In use, a liquid alloy of components (B) and (C) is formed in the inventive catalyst composition. This liquid alloy is present on said support material. These features distinguish the inventive catalyst compositions from known catalyst compositions, where the catalytic active material (C) is present in solid form. Without being bound to theory, it is believed that these features result in the improvements observed, namely higher reactivity, higher selectivity, less deactivation. In its broadest sense, the invention thus provides for the use of an alloy comprising components (B+C), as defined in claim 1, which is located on a support comprising component (A) in catalytic reactions, whereby said alloy is liquid under the conditions of said catalytic reaction. The inventive composition of matter thus benefits from the cooperative effect of components (A), (B) and (C). While (A) provides for the well-known function of a support and (C) provides for the well-known function of a catalyst site, component (B) provides for a liquid environment of the catalyst (C). It was unexpected, that the component (C) retains its catalytic function in such liquid environment, despite the teachings of the prior art which suggest the importance of crystalline structures (see e.g. Sattler et al, cited above, figs. 12 and 20). Moreover, and explained in further detail below, the catalysts become more active, more selective and show less deactivation when compared to the known catalysts.

This aspect of the invention shall be explained in further detail below:

**Support:** Support materials, also termed carriers, are generally known and may be selected by the skilled person according to the catalysed reaction. The support contains, i.e. comprises or consists of, component (A). Accordingly, such support materials may comprise promotors or other components to increase stability, or otherwise beneficially influence catalysis. Support materials are generally not catalytically active themselves.

The support can be formed in any suitable shape, such as a sphere, cylinder, tablet, powder and the like. In a preferred embodiment the catalyst carrier is a sphere or a cylinder. The spherical or cylindrical catalyst carrier can be formed in any suitable size, for a spherical catalyst carrier preferably from about 1 to about 30 millimetres in diameter. Alternatively, the support can be formed as a monolithic structures adapted to a specific housing, preferably with sizes of 10 cm or more in one dimension. Accordingly, the invention provides in advantageous embodiments for a composition as described herein, wherein said support is

- in the form of a powder, particularly with particle size in the range of 10 to 800 $\mu$m;
- in the form of granulated material ("granules"), such as pellets or beads, particularly with a particle size of 50 to 5000 $\mu$m;
- in the form of spheres, such as beads or cylinders, particularly with particle size of 0.5-50 mm such as 1 to 30 mm;
- in the form of a shaped article, such as a porous monolith, preferably with sizes of 10 cm or more in one dimension.

**Coating:** Components (B) and (C) are located on the surface of the support, thereby coating component (A). Without being bound to theory, it is believed that the liquid alloy comprising components (B) and (C) is adsorbed on

support (A) .

In one embodiment, components (B+C) fully cover said support, thereby forming a continuous film. The film thickness may vary, but typically is in the range of at least 0.4 nm. Maximum film thickness is determined by the loading of (B+C), support surface and total pore volume of the support and is considered less critical.

In one alternative embodiment, components (B+C) partly cover said support, thereby forming a discontinuous coating or droplets. It was found that catalyst compositions are suitable where at least 1 %, preferably at least 10 % of the support are covered with component (B+C).

**Component (A):** Suitable support materials (A) are selected from the group consisting of metal oxides, silicates, and carbonaceous materials. Such materials are known in the field and are commercially available or may be obtained according to known processes.

Metal oxides include alumina, silica-alumina, zinc oxide, nickel spinel, titania, zirconia, ceria, chromia-alumina, magnesium oxide, cerium oxide and mixtures thereof. The preferred metal oxide support is alumina.

Silicates include all salts and esters of Si(OH)4 and their condensates. These compounds are composed of SiO4 tetrahedrons that are connected among each other in various ways. Unconnected position in the structure of silicates carry alkali ions for charge compensation or form hydroxide terminations. The term silicates thus includes the parent compound SiO2. The preferred silicate support is a porous glass with a majority of its pores being above 2 nm pore size.

Carbonaceous materials include porous carbon materials obtained synthetically from pyrolysis of carbon containing precursors such as tar, coconut or organic polymers. These porous carbon materials may be activated by treatments with water vapour or carbon dioxide at elevated temperatures. The preferred carbonaceous support contains a pore size distribution that offers more than 5% of its pore volume in form of pores with a pore diameter larger than 2 nm.

Component (A) preferably is a high surface material (such as a porous material), to provide increased surface area for the catalytic reactions. Thus, the specific surface may vary over a broad range. Suitable support materials may have a specific surface area (SSA) of 2-10000 $m^2/g$, preferably 50-1000$m^2/g$. SSA may be determined according to BET methods.

Metal oxides, silicates and carbonaceous materials, as discussed herein, are typically considered as high surface area support materials for many catalytic reactions, in case the support has a surface area greater than about 150 m2/g, preferably from about 150 to about 1000 m2/g, more preferably from about 175 to about 500 m2/g, and most preferably from about 200 to about 300 m2/g. The pore volume of the metal oxides, silicates and carbonaceous materials, as discussed herein, may vary over a broad range, but is preferably in the range of about 0.2 to about 0.6 cc/g.

Component (A) preferably is thermally stable. Suitable support materials may be selected by the skilled person according to the catalysed reaction and are typically thermally stable up to at least 400°C, preferably up to at least 800°C.

**Component (B):** As component (B) gallium and its alloys are selected. It is understood that the terms gallium and gallium alloys relate to the oxidation state +/-0, thereby excluding metal salts and metal oxides. Component (B) has a melting point below 200°C. It is apparent that component (B) is selected not to inhibit catalytic activity of component (C). Suitable metals are selected from the group consisting of Ga (melting point= 30 °C) . Suitable alloys are selected from gallium-indium eutectics (melting point down to 15 °C).

In an advantageous embodiment, component (B) is gallium. In a further advantageous embodiment, component (B) is a gallium containing alloy, typically with more than 50% Ga; such as a gallium-indium alloy.

**Component (C):** As component (C), any metal suitable for a given catalytic reaction, and selected from the group of metals as defined by IUPAC group 3 to 12 and from the group of alloys comprising a metal as defined by IUPAC group 3 to 12, may be used. Preferred are metals (C) that dissolve in component (B) to at least 1 wt%, preferably at least 10wt% at temperatures above 200°C.

In the context of this invention, component (C) is catalytically active, if the reaction to be catalysed occurs at that component (C), or if component (C) induces catalytic properties in component (B). In other words, the catalytic mechanism is not relevant. Any component (C), which is either catalytically active or which promotes catalytic activity, may be used according to this invention.

Suitable components (C) are selected from the group of metals as defined by IUPAC group 3 to 12, particularly platinum group metals; and from the group of alloys comprising a metal as defined by IUPAC group 3 to 12, particularly alloys comprising platinum group metals.

In one embodiment, component (C) is selected from the group consisting of Ti, Zr, Cr, Mo, W, Rh, Fe, Ru, Co, Rh, Ir, Ni, Pd Pt, Cu, Ag, Au and its mixtures.

In a preferred embodiment, component (C) is selected from the group consisting of Pt, Pd, Ru, Rh, Ir, Ni, Fe, Cr, Cu, Co and its mixtures.

In a further preferred embodiment, component (C) is selected from the group alloys consisting of Pt/Co, Pt/Sn, Pt/Re, Pd/Zn.

In a particular preferred embodiment, component (C) is selected from Pd, Pt, Rh and Cr.

The amount of (B) and (C) present on the catalyst composition may vary over a broad range; suitable ranges

may be determined in routine experiments, dependent on the catalytic reaction and the reactor design. Typical ranges for the amount [(C)+(B)]:(A) are from about 0.01 to about 300 weight percent, preferably 0.05 to 50 weight percent, based on the total weight of the support.

The amount of (B) to (C) may also vary over a broad range; suitable ranges may be determined in routine experiments, dependent on the catalytic reaction and the reactor design. Typical ranges for the amount (B):(C) are from about 1 :1 to 1000:1, preferably 5:1 to 100: 1, such as 10:1 to 20:1.

In a **second aspect,** the invention relates to a process for manufacturing a composition as described herein. The components (B) and (C) can be introduced into the support (A) by any conventional procedure which produces the proper metal loading. Such methods are known per se but not yet applied to the specific combination of components (A), (B) and (C).

This aspect of the invention shall be explained in further detail below:
One preferred technique involves impregnating the support with a solution comprising a precursor of component (B) followed by solvent removal to obtain a coated material. This coated material is than subjected to a second impregnation step where a solution comprising a precursor of component (C) is contacted with the initially obtained coated material. After solvent removal, and optionally further activation steps, the inventive composition is obtained. Accordingly, the invention provides for a method for manufacturing a composition as described herein, comprising the steps of

(i) providing a component (A), a liquid composition comprising a precursor of component (B) and a liquid composition comprising a precursor of component(C);
(ii) contacting component (A) with said liquid composition of (B) and removing solvent;
(iii) contacting the thus obtained materials with said liquid composition of (C) and removing solvent;
(iv) optionally further activation steps;

to thereby obtain said solid composition of matter.

An alternative technique involves impregnation with a liquid composition comprising both, first precursor of (B) and second precursor of (C) in one single impregnation step. Accordingly, the invention provides for a method for manufacturing a composition as described herein, comprising the steps of

(i) providing a component (A) a liquid composition comprising a precursor of component (B) and of component (C);
(ii) contacting component (A) with said liquid compositions comprising (B) and (C) and removing solvent;
(iii) optionally further activation steps;

to thereby obtain said solid composition of matter.

Suitable precursors of component (B) and of component (C) are known and available by known methods; they are selected from the class of alkylammnonium gallates as precursor of component (B) and from the classes of alkylammonium halometallates and of ammonium halometalattes as precursor of component (B).

As outlined above, the compositions obtained according to the methods described herein have outstanding catalytic properties. The invention therefore also describes a solid composition, particularly a catalyst composition, obtained by a method as described herein.

In a **third aspect,** the invention relates to the use of compositions as described herein, particularly to catalytic reactions employing the compositions described herein. It has been surprisingly discovered that high performing catalysts, particularly selective dehydrogenation catalyst, are obtained when a combination of components (B) and (C) is coated on a high surface area inert support (A). Unexpectedly, the inventive catalyst systems are less prone to coking, thereby showing less deactivation. This allows for reduced regeneration times and longer reaction times when compared to known catalyst compositions.

The inventive use introduces a new paradigm in catalysis, namely supported catalytically active liquid metal solutions (SCALMS). Conversely to traditional supported liquid phase catalysis, it is believed that the catalytic reaction here takes place at the substrate-liquid metal interface, as the liquid metal phase provides no solubility for organic reactants. At the same time, however, the liquid metal solution film is highly dynamic and the catalytic reactions proceeds at the isolated homogeneously distributed active metal atoms at the surface of the liquid metallic phase. In a more general sense, supporting a dissolved catalytic active metal in a metallic solution merges some features of heterogeneous catalysis, i.e. easy product separation, with features of homogeneous catalysis, i.e. electronic / steric control, due to the formation of isolated metal sites in a certain metal matrix, which in turn present different electronic features due to the intermetallic interactions. This mechanistic considerations apply to a broad range of catalytic reactions.

This aspect of the invention shall be explained in further detail below:
In use, the inventive catalyst composition is placed in a reactor. The inlet temperature of the feed stream in the reactor is raised to a level sufficient to perform the reaction and is above the melting temperature of component (B). Generally, this temperature is above 200°C. Further, the temperature is chosen to ensure integrity of the support material. This

temperature depends on the support, but typically is below 1500°C, such as below 1000°C.

In one embodiment, the invention provides for the use of a composition as described herein (first aspect), as a catalyst, particularly as a catalyst in a gas-phase reaction. In such gas-phase reactions, any suitable reaction pressure can be used. Generally, the total pressure in the reactor is in the range between 1 bar and 100 bar, preferably between 1 bar and 30 bar. In such gas-phase reactions, the gas hourly space velocity (GHSV) may vary over a broad range, but typically is of about 100 to about 100.000 liters per liter of catalyst per hour.

In one embodiment, the invention provides for the use of a composition as described herein (first aspect), where said catalysed reaction is selected from the group of endothermic reactions. Endothermic reactions are typically performed at higher temperatures, to beneficially influence the reaction equilibrium. Such high temperatures in turn favour side reactions, such as coke deposition on the catalyst. The inventive catalyst system allows higher reaction temperatures while still avoiding coke formation and thereby deactivation of catalyst. As a result, reaction cycles may be more than 1 h, preferably more than 100 h before regeneration becomes necessary.

Accordingly, the invention provides for a catalytic process, comprising the step of contacting an organic starting material with a composition described herein (first aspect), where

- reaction temperatures are above 200°C; and/or
- reaction cycles are at least 1 hour; and/or
- starting materials and reaction products are gaseous under reaction conditions.

A broad range of catalytic reactions may benefit from the inventive catalyst compositions. Due to the high reaction temperatures, endothermic reactions are preferred. Endothermic reactions shall include both (i) reactions where all reaction steps are endothermic and (ii) reactions where some reaction steps are exothermic but the over all reaction being endothermic. Moreover, slightly exothermic reactions may still benefit from the inventive catalytic systems and are thus also included. Preferably the catalytic process described herein is selected from the group of

- catalytic isomerisation of hydrocarbons or of aromatics,
- catalytic dehydrogenation of hydrocarbons,
- catalytic reforming.

These catalytic processes are known per se but not yet performed with the catalyst compositions described herein.

Preferred feedstocks for the above described catalytic processes include aliphatic and aromatic hydrocarbons, including C2+ alkanes (such as linear, branched and cyclic C2-24 alkanes) and C6+ aromatics, such as C6-14 arenes and C6-C14 arenes subsstitued with one or more C1-4 alkyl groups). Specific feedstocks include ethane, propane, butane, isobutane, pentane, isopentanes, hexane, isohexanes, cyclohexane, heptane, isoheptanes, methylcyclohexane, ethyl benzene, perhydro benzyltoluene, perhydro dibenzyltoluene and mixtures thereof.

A particularly preferred reaction is the dehydrogenation of hydrocarbons, particularly of the hydrocarbons cited above. As discussed above, existing catalysts do not perform consistently well, particularly in terms of conversion rate, selectivity and stability. By the process of this invention, these issues are successfully addressed as outlined in the examples and supported by the figures 1-9. Specifically, long-term stability over more than 100 h is observed (fig. 7) while the prior art reports stability of commercial catalysts being excellent when regeneration becomes necessary after 7 h (Sattler, cited above, chapter 2.3). Further, catalytic activity (indicated as TOF) and selectivity of the inventive catalyst composition is consistently higher compared to the commercial catalysts (figs. 2-5).

Regeneration of the inventive catalyst composition, once required, may be accomplished according to standard procedures, e.g. by heating the catalyst composition in air or hydrogen, to thereby burn off any deposited material, such as coke.

The amount of catalyst used for a specific catalytic reaction may vary over a broad range. Typically, the amount of the inventive catalyst for a given catalytic reaction is in the same range or lower as the amount of catalyst currently used in such reactions.

Without being bound to theory, it is believed that at least three cooperating effects provide for the excellent catalytic properties disclosed herein.

First, the liquid nature of alloy (B+C), as present under reaction conditions, provides for a highly dynamic behaviour on the fluid-fluid interface and a continuous regeneration of the alloy surface under reaction conditions. This highly dynamic behaviour is likely to supresses coking.

Second, component (C), although present in small amounts, provides for a high catalytic activity. This may be attributed to an enrichment of (C) on the liquid alloy surface and thereby facilitates contact to the feedstock. As a third effect, the high surface area provided by the support and the low vapour pressure of the alloy (B+C) provides for a large contact area of feedstock to catalytic active material.

To further illustrate the invention, the following **examples** are provided. These examples are provided with no

intend to limit the scope of the invention.

## 1. Catalyst Preparation

### 1.1 Pd/Ga decorated material

**Preparation of $(Et_3N)GaH_3$**

This compound was synthetized using the procedure described by Shriver et Al. (Inorg. Synth. 1977, 17, 42-45) using triethylammonium chloride (Sigma-Aldrich) instead of trimethylammonium. The compound was not isolated and used as ethereal solution.

**Preparation of $(NH_4)_2[PdCl_4]$**

A 10 mM solution of $(NH_4)_2[PdCl_4]$ was prepared as follows: $PdCl_2$ (171.5 mg, 1 mmol, Sigma-Aldrich) and $NH_4Cl$ (108.3 mg, 2 mmol, Sigma-Aldrich) were added to ca. 30 mL of distilled water in a beaker. The resulting suspension was boiled until it became homogeneous, then cooled and transferred to a 100 mL calibrated flask and diluted with distilled water.

**Preparation of Ga decorated porous glass**

10 g of porous glass (non-treated SiO2, available as trisopor, Biosearch Technologies) were heated under vacuum (1 mbar) at 300 °C overnight in a Schlenk flask. After cooling the system, they were suspended in 20 mL dry diethyl ether, under argon. To this suspension was added an ethereal solution of $Et_3NGaH_3$ in such amount to obtain a theoretical Ga loading of 5 % w/w of Ga with respect to the support. The ether was removed under vacuum at ca. -30 °C. After complete removal of diethyl ether, the flask was quickly (ca. 10 °C /min) heated up to 250 °C and held at this temperature until no gaseous products were developed. Once the gallane decomposition terminated, the resulting grey solid was held at this temperature under vacuum (1 mbar) overnight and then cooled and stored under argon to thereby obtain the tile compound.

**Preparation of Pd/Ga decorated material**

2 g of Ga decorated porous glass were suspended in 10 mL of distilled water under vigorous stirring. To this suspension was added a stock solution of $NH_4[PdCl_4]$ (with a concentration of 2 mg $mL^{-1}$) in distilled water, in such amount to get the desired theoretical Ga/Pd ratio, once accounted for the loss of Ga due to the redox reaction between the latter and the $[PdCl_4]^{2-}$ anion. The resulting suspension was held under stirring until the reaction completed (few seconds: the orange solution turns colorless), then filtered and thoroughly washed in turn with distilled water (250 mL) and acetone (150 mL). The resulting solid was first roughly air dried and then under vacuum (1 mbar) overnight.

### 1.2 Preparation of Pt/Ga, Rh/Ga and Cr/Ga decorated materials

Triethylammonium chlorometallates of the general formula $[Et_3NH]_n[MCl_{m+n}]$ were chosen as metal precursors. These compounds were prepared according the scheme depicted below, by mixing the relevant metal chloride (Sigma-Aldrich) with a stoichiometric amounts of $[Et_3NH]Cl$ (Sigma-Aldrich) and refluxing the mixture in acetonitrile or acetonitrile/MeOH mixtures until complete dissolution:

$$n\,[Et_3NH]Cl + MCl_m \xrightarrow[\substack{CH_3CN\\ \text{or}\\ CH_3CN/MeOH}]{} [Et_3NH]_n[MCl_{m+n}]$$

n=m=2; **M** = Pt, Ni, Cu, Co
n=1; m=3; **M** = Au, Fe, Cr, Ir, Rh, Ru

The solutions were prepared in order to obtain 0.1 g of active metal. To these metal precursor solutions, porous glass (10.0 g) was added and the solvent removed by mean of a rotavapor. The resulting solids were dried overnight at 80 °C under vacuum (0.5 mbar).

After this treatment, the obtained solids were suspended in dry ethyl ether (20 mL). The M/Ga catalyst were

prepared by adding to the suspensions a 2 M solution of $Et_3NGaH_3$ in diethyl ether, in such amount to realize a final M/Ga ratio of 10 mol/mol, once accounted for the consumed $Et_3NGaH_3$ due to the following reduction reaction:

$$[Et_3NH]_n[MCl_{n+m}] + m/3\ Et_3NGaH_3 \xrightarrow[Et_2O]{}$$

$$\xrightarrow[Et_2O]{} M^0 + (n \times m)/3\ [Et_3NH][GaCl_4] + m/2\ H_{2(g)} + m/3\ Et_3N$$

An immediate reaction took place, due to the reduction (darkening of solids) of the metals. The systems were left to react for 30 min. Afterwards the ether solution was slowly evaporated in vacuum at -30 °C in order to minimize the loss of the excess of volatile $Et_3NGaH_3$. When the solids were dry, they were heated up to 180 °C and kept at this temperature until no gas evolution was observed any longer. Then the temperature was raised up to 350 °C and the material was held at this temperature in a light Ar stream for 4 h. Then the Ar was switched off and the system held for further 2 h under vacuum (0.5 mbar). Then, the heating was turned off and the material was allowed to cool to room temperature to thereby obtain the M (Pt, Rh, Cr) / Ga decorated materials.

## 2. Catalytic reactions testing

### 2.1 setup

The catalysts are used as prepared according to the above procedure and are not reduced prior to catalytic testing. The catalytic tests were carried out in a continuous flow experimental plant at atmospheric pressure, which is depicted in figure 10. Depending on the metal loading a defined catalyst mass of 0.6 - 2.4 g is placed into the fixed-bed reactor. After a heating period of 2.5 h up to a temperature at the catalyst bed of 445 °C the reaction is started by supplying 9.3 $mL_N\ min^{-1}$ pure n-butane (Linde, purity 2.5) as feed gas, or 9.3 $mL_N\ min^{-1}$ n-butane diluted by 93 $mL_N\ min^{-1}$ helium (Linde, purity 4.6) respectively, resulting in a weight hourly space velocity (WHSV) of 60 - 255 $g_{n\text{-butane}}\ g_{Pd}^{-1}\ h^{-1}$ and a residence time $\tau$ of 0.3 - 7.3 s.

The gases are dosed by mass flow controllers (MFC, Bronckhorst). All reactor tubes and pipes are heated by the use of heating tapes to a temperature of 180 °C and are isolated with fiberglass tape. The Alloy 600 fixed-bed reactor is heated by a heating jacket which is divided into three parts. All other parts which are in contact with the reactants are made of stainless steel 1.4571.

### 2.2 Analytic techniques

### GC

The product stream is analyzed by an online Bruker 450 GC equipped with a ShinCarbon micropacked CP Wax 52 CB column (25 m x 0.53 mm x 0.7 mm), a HP-AL/S column (50 m x 0.535 mm x 0.015 mm) and a flame ionization detector (FID) . Required mole fractions x are calculated from peak areas and calibration factors determined for every substance. The conversion of n-butane $X_{n\text{-butane}}$, turn over frequency (TOF) with regard to n-butane and selectivity to butenes $S_{butenes}$ (as the sum of 1-butene, cis-2-butene, trans-2-butene, isobutene) are calculated as follows:

$$X_{n-butane} = \frac{x_{n-butane,0} - x_{n-butane}}{x_{n-butane,0}} \cdot 100\ \% \qquad (1)$$

$$TOF = \frac{\dot{n}_{n-butane,0} \cdot X_{n-butane}}{n_{active\ Metal}} \qquad (2)$$

$$S_{butenes} = \frac{x_{butenes} - x_{butenes,0}}{x_{n-butane,0} - x_{n-butane}} \cdot 100\ \% \qquad (3)$$

The corrected conversion of n-butane $X^*_{n\text{-butane}}$ is defined as the measured conversion of the catalytic test reduced by the blank activity of the reactor $X_{n\text{-butane},0}$. The corrected turn over frequency (TOF*) is calculated analogous to the TOF using the corrected conversion X* instead of X.

**ICP-AES**

The content of active metal and gallium of the catalyst samples was measured by inductively coupled plasma - atom emission spectroscopy (ICP-AES) using a Ciros CCD (Spectro Analytical Instruments GmbH). The solid samples were digested in HCl:$HNO_3$:HF ratio 3:1:1 in a microwave oven at 180 °C for 40 min and the instrument was calibrated with standard solutions of the relevant substances prior to the measurements.

**2.3 Catalytic experiments**

Table 1 shows the composition of a Pd/Ga catalyst for catalytic experiments, obtained according to the procedure described above.

Table 1

| | $n_{Ga}/n_{Pd}$ | mol% Ga | mol% Pd | wt%Ga | wt% Pd |
|---|---|---|---|---|---|
| diluted | **10** | 90 | 10 | 2.4 | 0.4 |
| diluted | **20** | 95 | 5 | 4. 9 | 0.4 |
| non. diluted | **10.8** | 92 | 8 | 3.4 | 0.5 |
| non. diluted | **22.8** | 96 | 4 | 3.5 | 0.2 |

Table 2 shows the composition of Rh/Ga, Pt/Ga and Cr/Ga catalysts for catalytic experiments with undiluted butane feed, obtained according to the procedure described above.

Table 2

| Metal | $n_{Ga}/n_{Metal}$ | mol% Ga | mol% Metal | wt%Ga | wt% Metal |
|---|---|---|---|---|---|
| Rh | 9.2 | 90 | 10 | 4.9 | 0.8 |
| Pt | 8.4 | 89 | 11 | 2.8 | 0.9 |
| Cr | 4.6 | 82 | 18 | 5.6 | 0.9 |

**Diluted experiments with the Pd/Ga catalyst:**

The following parameters were used for diluted experiments: $T_{jacket}$ = 500 °C, $T_{cat,m}$ = 445 °C, p = 1,1 bar, V · butane = 9,3 $mL_N$ $min^{-1}$, V · He = 93 mLN $min^{-1}$, $m_{cat}$ = 0,6 - 1,6 g, $m_{Pd,rct}$ = 0,006 g, WHSV = 255 $g_{butane}$ $g_{Pd}^{-1}$ $h^{-1}$.

The results being shown in figs. 1-3. Fig. 1 shows conversion of n-butane (%) over time (h). Fig. 2 shows the corresponding TOF* ($h^{-1}$) (TOF*: TOF corrected by blank value of reactor). Fig. 3. shows the corresponding selectivity to butenes (%).

In each of figs. 1-3 the symbols have the following meaning: a "-" represents the empty reactor, an "x" the support, an empty diamond (or triangle) Pd-coated support, an empty square the Ga-coated support. These data are included for comparison. The experiments with the inventive catalyst are marked with solid symbols: Ga/Pd=10 as solid triangle and Ga/Pd=20 solid circle.

This experiment clearly shows the superior properties of the inventive catalyst. Specifically, the combination of components A, B, and C results, in the catalytic experiments described, a significant and commercially relevant increase of catalytic activity (TOF), selectivity and life expectancy.

**Non-diluted experiments with Metal (Pd, Rh, Pt, Cr) /Ga catalyst::**

The following parameters were used for non-diluted experiments: $T_{jacket}$ = 500 °C, $T_{cat,m}$ = 445 °C, p = 1,1 bar, V · butane = 9,3 $mL_N$ $min^{-1}$, $m_{cat}$ = 1 - 1,4 g, $m_{metal,rct}$ = 0,006 - 0,049 g, WHSV = 60 - 255 $g_{butane}$ $g_{Pd}^{-1}$ $h^{-1}$.

The results being shown in figs. 4 and 5 for the Pd/Ga decorated material. Figs 8 and 9 show results for Rh/Ga, Pt/Ga and Cr/Ga decorated materials.

Fig. 4 shows the TOF* ($h^{-1}$) (TOF*: TOF corrected by blank value of reactor). Fig. 5 shows the corresponding selectivity to butenes (%). In each of figs. 4 and 5 an empty triangle indicates the Pd-coated support. The experiments with the inventive catalyst are marked with solid symbols: Ga/Pd=10.8 as solid triangle and Ga/Pd=22.8 solid circle.

Fig. 8 shows the TOF* ($h^{-1}$) (TOF*: TOF corrected by blank value of reactor). Fig. 9 shows the corresponding

selectivity to butenes (%). In both figs, a solid circle represents Rh/Ga, a solid triangle represents Pt/Ga and a solid square represents Cr/Ga.

As for the diluted experiment, this non-diluted experiment clearly shows the superior properties of the inventive catalyst. Specifically, the combination of components A, B, and C results, in the catalytic experiments described, a significant and commercially relevant increase of catalytic activity (TOF), selectivity and lifetime. It is particularly to be noted that the inventive catalyst system also performs excellent under the above-described, non-diluted conditions. The prior art, e.g. as cited above, typically reports on diluted experiments. Such reports typically provide better results in a lab-scale but are of less relevance when considering commercial applications. Further, as evidenced by figs. 8 and 9, a broad variety of metals retain or improve its catalytic activity, selectivity and stability and may therefore be used as component C in the inventive catalytically active materials.

**Comparison to commercial catalysts (non-diluted)**

The following parameters were used for comparative experiments: $T_{jacket}$ - *500 °C*, $T_{cat,m}$ *= 445 °C, p = 1,1 bar, V · butane = 9,3 mLN* $min^{-1}$, $m_{Kat}$ *= 1 - 1,4 g,* $m_{met,rct}$ *= 0,006 -0,049 g, WHSV = 60 - 255* $g_{butane}$ $g_{Pd}^{-1}$ $h^{-1}$.

Fig 6 compares the TOF* (h-1) vs. time (h) of the inventive catalyst with commercially available catalysts. Empty circle and empty triangle represent commercial CrAlOx- and Pt/AlOx-dehydriation catalysts respectively, while solid squares represent the inventive catalyst Ga/Pd= 10.

The same chromium oxide catalyst to which the performance of the inventive catalysts is compared, is also published as it was used for a cascade reaction combining dehydrogenation and hydroformylation by Walter et al. [S. Walter et al., American Institute of Chemical Engineers Journal, 2015, 61, 893-897].

The superior properties of the inventive catalytically active material are apparent.

**Long-term stability (non-diluted)**

The following parameters were used for the long term experiment: $T_{jacket}$ *= 500 °C*, $T_{cat,m}$ *= 445 °C, p = 1,1 bar, V · butane = 9,3* $mL_N$ $min^{-1}$, $m_{cat}$ *= 1,2 g,* $m_{Pd,rct}$ *= 0,006 g, WHSV = 255 gbutane* $g_{Pd}^{-1}$ $h^{-1}$

The results being shown in figs. 7 where TOF* (h-1) vs. time (h) of the inventive catalyst Ga/Pd= 10 is shown. This experiment clearly shows long-term stability of the inventive catalyst.

## Claims

**1.** A solid composition of matter, consisting of a support and a coating, whereby said support comprises a non-metallic, porous component (A), and said coating consists of a first metallic component (B) and a second metallic component (C), **characterized in that**

- said component (A) is selected from the group consisting of metal oxides, silicates, and carbonaceous materials,
- said component (B) is selected from the group consisting of gallium and gallium containing alloys having the oxidation state +/-0 and having a mp. below 200°C,
- said component (C) is catalytically active and selected from the group of metals as defined by IUPAC group 3 to 12 and from the group of alloys comprising a metal as defined by IUPAC group 3 to 12,
- said component (C) is present in component (B) in dissolved form,
- said component (C) has a solubility of at least 0.01 wt-% in component (B),
- said coating has a melting point below 200°C.

**2.** The composition of claim 1 where said component (A)

- has a specific surface of 2-10000 m2/g; and / or
- is thermally stable up to 400°C.

**3.** The composition according to any of the preceding claims, where said component (A) is selected from the group consisting of aluminium oxide and silica; and / or said component (B) is a metal selected from the group consisting of Ga or an alloy selected from the group consisting of gallium - indium eutectics; and / or said component (C) selected from the group consisting of Pd, Pt, Rh, and Cr; and from the group of alloys comprising Pt/Co, Pt/Sn, Pt/Re, and Pd/Zn.

4. The composition according to any of the preceding claims, wherein said support is

- in the form of a powder;
- in the form of granulated material;
- in the form of spheres; or
- in the form of a shaped article.

5. The composition according to any of the preceding claims, wherein said coating

- fully covers said support, preferably with a film thickness of at least 0.4 nm; or
- partly covers said support, preferably to more than 1 Area-%.

6. The composition according to any of the preceding claims, wherein the amount of [(C)+(B)]:(A) is from about 0.01 to about 300 wt.% based on the total weight of the support.

7. A method for manufacturing a composition according to any of the preceding claims, comprising the steps of

(i) providing a component (A); a solution comprising a precursor of (B) selected from the group of alkylammonium gallates and a solution comprising a precursor of (C) selected from the group of alkylammonium or ammonium halometallates;
(ii) contacting said component (A) with said solutions comprising precursors of (B) and (C) either simultaneously or subsequently, removing solvent; and
(iii) optionally further activation steps;

to thereby obtain said solid composition of matter.

8. The method of claim 7 wherein

- said precursor of (B) is Et3NGaH3 and
- wherein said precursor of (C) is selected from $[Et_3NH]_n[MCl_{m+n}]$ and

  m=n=2 and M= Pt, Ni, Cu, Co; or
  m=3, n=1, M= Au, Fe, Cr, Ir, Rh, Ru.

9. Use of a composition according to any of claims 1 - 6 as a catalyst, particularly as a catalyst in a gas-phase reaction.

10. The use according to claim 10, where said catalysed reaction is selected from the group of endothermic reactions.

11. A catalytic process, comprising the step of contacting an organic starting material with a composition according to any of claims 1 - 6, where

- reaction temperatures are above 200°C; and/or
- reaction cycles are at least 1 hour; and/or
- starting materials and reaction products of the catalytic process are gaseous under reaction conditions.

12. The catalytic process according to claim 12, said process being selected from the group of

- catalytic isomerisation of hydrocarbons or of aromatics,
- catalytic dehydrogenation of hydrocarbons,
- catalytic reforming.

13. The catalytic process according to claims 12 and 13, the starting material being selected from the group consisting of aliphatic or aromatic hydrocarbons, including linear, branched and cyclic C2+ alkanes or C6+ aromatics, and mixtures thereof.

## Patentansprüche

1. Eine feste Stoffzusammensetzung, bestehend aus einem Träger und einer Beschichtung, wobei der Träger eine nichtmetallische, poröse Komponente (A) umfasst, und die Beschichtung eine erste metallische Komponente (B) und eine zweite metallische Komponente (C) umfasst, **dadurch gekennzeichnet, dass**

   - die Komponente (A) aus der Gruppe ausgewählt wird, die aus Metalloxiden, Silikaten und kohlenstoffhaltigen Materialien besteht,
   - die Komponente (B) aus der Gruppe ausgewählt ist, die aus Gallium und galliumhaltigen Legierungen mit der Oxidationsstufe +/-0 und mit einem mp. unter 200°C besteht,
   - die Komponente (C) katalytisch aktiv ist und ausgewählt ist aus der Gruppe der Metalle, wie durch die IUPAC-Gruppe 3 bis 12 definiert, und aus der Gruppe der Legierungen, die ein Metall, wie durch die IUPAC-Gruppe 3 bis 12 definiert, umfassen,
   - die Komponente (C) in der Komponente (B) in gelöster Form vorliegt,
   - die Komponente (C) eine Löslichkeit von mindestens 0,01 Gew.-% in Komponente (B) aufweist,
   - die Beschichtung einen Schmelzpunkt unter 200°C hat.

2. Die Zusammensetzung nach Anspruch 1, wobei die Komponente (A)

   - eine spezifische Oberfläche von 2-10000 m2/g hat; und / oder
   - thermisch stabil bis zu 400°C ist.

3. Die Zusammensetzung nach einem der vorangehenden Ansprüche, wobei die Komponente (A) ausgewählt ist aus der Gruppe bestehend aus Aluminiumoxid und Siliciumdioxid; und / oder die Komponente (B) ein Metall ist, das aus der Gruppe ausgewählt ist, die aus Ga oder einer Legierung besteht, die aus der Gruppe bestehend aus Gallium-Indium-Eutektika ausgewählt ist; und / oder wobei die Komponente (C) aus der Gruppe ausgewählt ist, die aus Pd, Pt, Rh und Cr besteht; und aus der Gruppe von Legierungen, die Pt/Co, Pt/Sn, Pt/Re und Pd/Zn umfassen.

4. Die Zusammensetzung nach einem der vorangehenden Ansprüche, wobei der Träger

   - in Form eines Pulvers;
   - in Form von granuliertem Material;
   - in Form von Kugeln; oder
   - in Form eines geformten Artikels vorliegt.

5. Die Zusammensetzung nach einem der vorangehenden Ansprüche, wobei die Beschichtung

   - den Träger vollständig bedeckt, vorzugsweise mit einer Schichtdicke von mindestens 0,4 nm; oder
   - den Träger teilweise bedeckt, vorzugsweise auf mehr als 1 Gebiets-%.

6. Die Zusammensetzung nach einem der vorangehenden Ansprüche, wobei die Menge von [(C)+(B)]:(A) von etwa 0,01 bis etwa 300 Gew.-%, bezogen auf das Gesamtgewicht des Trägers, beträgt.

7. Ein Verfahren zur Herstellung einer Zusammensetzung nach einem der vorangehenden Ansprüche, umfassend die folgenden Schritte

   (i) Bereitstellen einer Komponente (A); einer Lösung, die einen Vorläufer von (B), ausgewählt aus der Gruppe der Alkylammoniumgallate, umfasst, und einer Lösung, die einen Vorläufer von (C), ausgewählt aus der Gruppe der Alkylammonium- oder Ammoniumhalometallate, umfasst;
   (ii) Inkontaktbringen der Komponente (A) mit den Lösungen, die Vorläufer von (B) und (C) umfassen, entweder gleichzeitig oder nacheinander, wobei Lösungsmittel entfernt wird; und
   (iii) optional weitere Aktivierungsschritte; um dadurch die feste Stoffzusammensetzung zu erhalten.

8. Das Verfahren nach Anspruch 7, wobei

   - der Vorläufer von (B) Et3NGaH3 ist und

wobei der Vorläufer von (C) ausgewählt ist aus
[Et3NH]n[MClm+n] und
m=n=2 und M= Pt, Ni, Cu, Co; oder
m=3, n=1, M= Au, Fe, Cr, Ir, Rh, Ru.

**9.** Verwendung einer Zusammensetzung nach einem der Ansprüche 1 - 6 als Katalysator, insbesondere als Katalysator in einer Gasphasenreaktion.

**10.** Die Verwendung nach Anspruch 10, wobei die genannte katalysierte Reaktion aus der Gruppe der endothermen Reaktionen ausgewählt wird.

**11.** Ein katalytisches Verfahren, umfassend den Schritt des Kontaktierens eines organischen Ausgangsmaterials mit einer Zusammensetzung nach einem der Ansprüche 1 bis 6, wobei

- die Reaktionstemperaturen über 200°C liegen; und/oder
- die Reaktionszyklen mindestens 1 Stunde betragen; und/oder
- die Ausgangsstoffe und Reaktionsprodukte des katalytischen Prozesses unter Reaktionsbedingungen gasförmig sind.

**12.** Das katalytische Verfahren nach Anspruch 12, wobei das Verfahren ausgewählt ist aus der Gruppe von
katalytische Isomerisierung von Kohlenwasserstoffen oder von Aromaten,
katalytische Dehydrierung von Kohlenwasserstoffen,
katalytische Reformierung.

**13.** Das katalytisches Verfahren nach den Ansprüchen 12 und 13, wobei das Ausgangsmaterial ausgewählt ist aus der Gruppe bestehend aus aliphatischen oder aromatischen Kohlenwasserstoffen, einschliesslich linearen, verzweigten und zyklischen C2+-Alkanen oder C6+-Aromaten, und ihren Mischungen.

## Revendications

**1.** Une composition solide de matière, constituée d'un support et d'un revêtement, dans laquelle
ledit support comprend un composant non métallique et poreux (A), et
ledit revêtement est constitué d'un premier composant métallique (B) et d'un second composant métallique (C),
**caractérisé en ce que**

- ledit composant (A) est choisi dans le groupe constitué par les oxydes métalliques, les silicates et les matières carbonées,
- ledit composant (B) est choisi dans le groupe constitué par le gallium et les alliages contenant du gallium ayant l'état d'oxydation +/-0 et ayant un mp. inférieur à 200°C,
- ledit composant (C) est catalytiquement actif et choisi dans le groupe des métaux tels que définis par les groupes 3 à 12 de l'UICPA et dans le groupe des alliages comprenant un métal tel que défini par les groupes 3 à 12 de l'UICPA,
- ledit composant (C) est présent dans le composant (B) sous forme dissoute,
- ledit composant (C) a une solubilité d'au moins 0,01 % en poids dans le composant (B),
- ledit revêtement a un point de fusion inférieur à 200°C.

**2.** La composition selon la revendication 1, dans laquelle ledit composant (A)

- a une surface spécifique de 2-10000 m2/g ; et / ou
- est thermiquement stable jusqu'à 400°C.

**3.** La composition selon l'une des revendications précédentes, dans laquelle
ledit composant (A) est choisi dans le groupe constitué par l'oxyde d'aluminium et la silice ; et / ou
ledit composant (B) est un métal choisi dans le groupe constitué par Ga ou un alliage choisi dans le groupe constitué par les eutectiques gallium - indium ; et / ou
ledit composant (C) est choisi dans le groupe constitué par Pd, Pt, Rh et Cr ; et dans le groupe des alliages comprenant Pt/Co, Pt/Sn, Pt/Re et Pd/Zn.

**4.** La composition selon l'une quelconque des revendications précédentes, dans laquelle ledit support est

- sous forme de poudre ;
- sous forme de granulés ;
- sous forme de sphères ; ou
- sous forme d'un article façonné.

**5.** La composition selon l'une des revendications précédentes, dans laquelle ledit revêtement

- couvre entièrement ledit support, de préférence avec une épaisseur de film d'au moins 0,4 nm ; ou
- couvre partiellement ledit support, de préférence à plus de 1 % de la superficie.

**6.** La composition selon l'une des revendications précédentes, dans laquelle le montant de [(C)+(B)] : (A) est d'environ 0,01 à environ 300 % en poids sur la base du poids total du support.

**7.** Un procédé de fabrication d'une composition selon l'une quelconque des revendications précédentes, comprenant les étapes suivantes

(i) fournir un composant (A) ; une solution comprenant un précurseur de (B) choisi dans le groupe des gallates d'alkylammonium et une solution comprenant un précurseur de (C) choisi dans le groupe des halométallates d'alkylammonium ou d'ammonium ;
(ii) mettre en contact ledit composant (A) avec lesdites solutions comprenant des précurseurs de (B) et (C) soit simultanément, soit ultérieurement, en éliminant le solvant ; et
(iii) éventuellement d'autres mesures d'activation ;

pour obtenir ainsi ladite composition solide de la matière.

**8.** Le procédé selon la revendication 7, dans laquelle

- ledit précurseur de (B) est l'Et3NGaH3 et
- ledit précurseur de (C) est choisi parmi $[Et_3NH]_n[MCl_{m+n}]$ et
m=n=2 et M= Pt, Ni, Cu, Co ; ou
m=3, n=1, M= Au, Fe, Cr, Ir, Rh, Ru.

**9.** Utilisation d'une composition selon l'une des revendications 1 à 6 comme catalyseur, en particulier comme catalyseur dans une réaction en phase gazeuse.

**10.** L'utilisation selon la revendication 10, dans laquelle ladite réaction catalysée est choisie dans le groupe des réactions endothermiques.

**11.** Un procédé catalytique, comprenant l'étape consistant à mettre en contact une matière première organique avec une composition selon l'une quelconque des revendications 1 à 6, où

- les températures de réaction sont supérieures à 200°C ; et/ou
- les cycles de réaction sont d'au moins 1 heure ; et/ou
- les matières premières et les produits de réaction du processus catalytique sont gazeux dans les conditions de la réaction.

**12.** Le procédé catalytique selon la revendication 12, ledit procédé étant choisi dans le groupe

- l'isomérisation catalytique des hydrocarbures ou des aromatiques,
- la déshydrogénation catalytique des hydrocarbures,
- le reformage catalytique.

**13.** Procédé catalytique selon les revendications 12 et 13, la matière première étant choisie dans le groupe constitué par les hydrocarbures aliphatiques ou aromatiques, y compris les alcanes en C2+ linéaires, ramifiés et cycliques ou les aromatiques en C6+, et leurs mélanges.

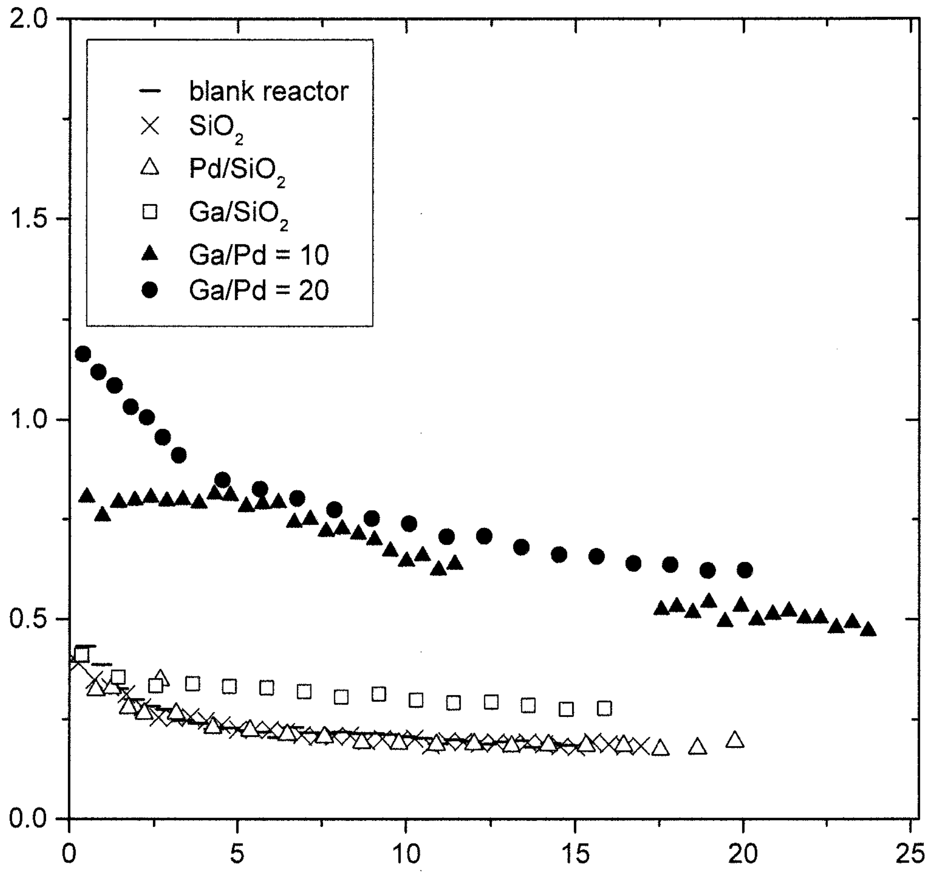
blank reactor
$SiO_2$
$Pd/SiO_2$
$Ga/SiO_2$
Ga/Pd = 10
Ga/Pd = 20
2.0
1.5
1.0
0.5
0.0
0
5
10
15
20
25

fig. 1

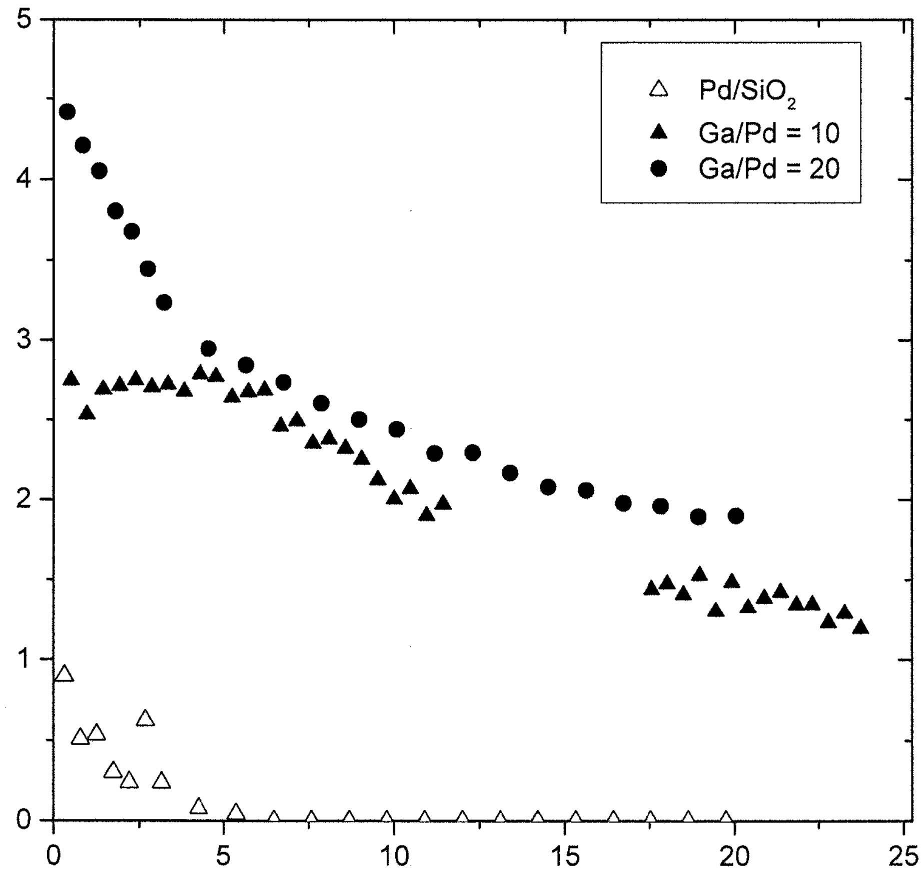
$Pd/SiO_2$
Ga/Pd = 10
Ga/Pd = 20
5
4
3
2
1
0
0
5
10
15
20
25

fig. 2

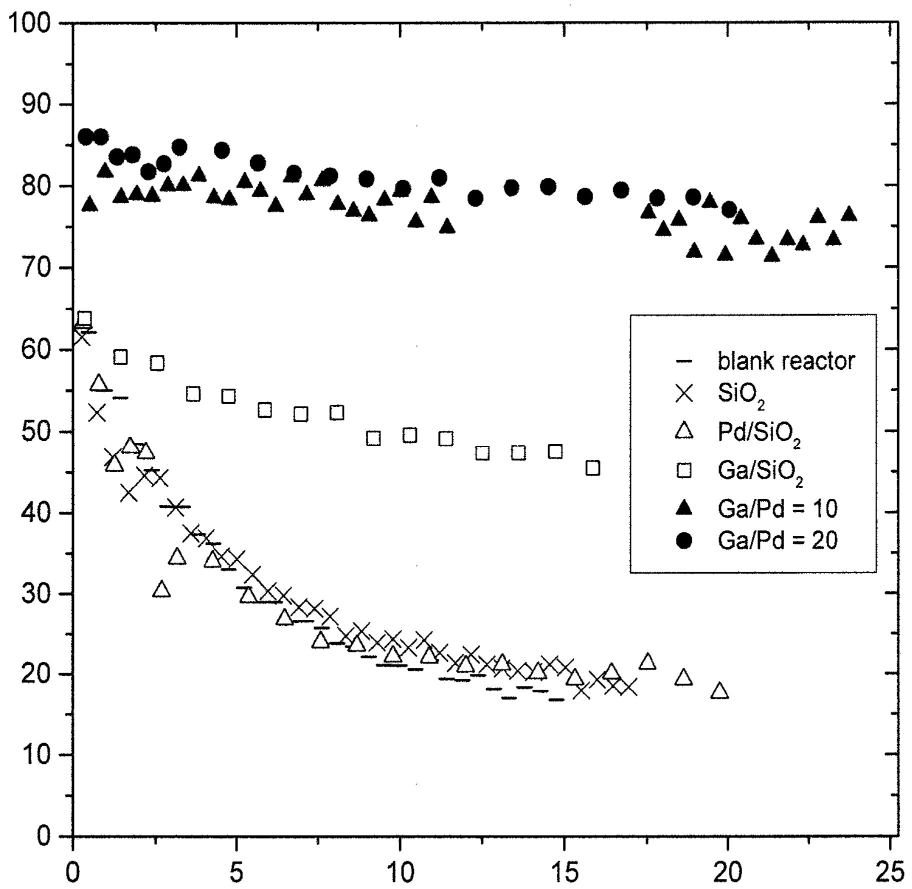
blank reactor
$SiO_2$
$Pd/SiO_2$
$Ga/SiO_2$
Ga/Pd = 10
Ga/Pd = 20

fig. 3

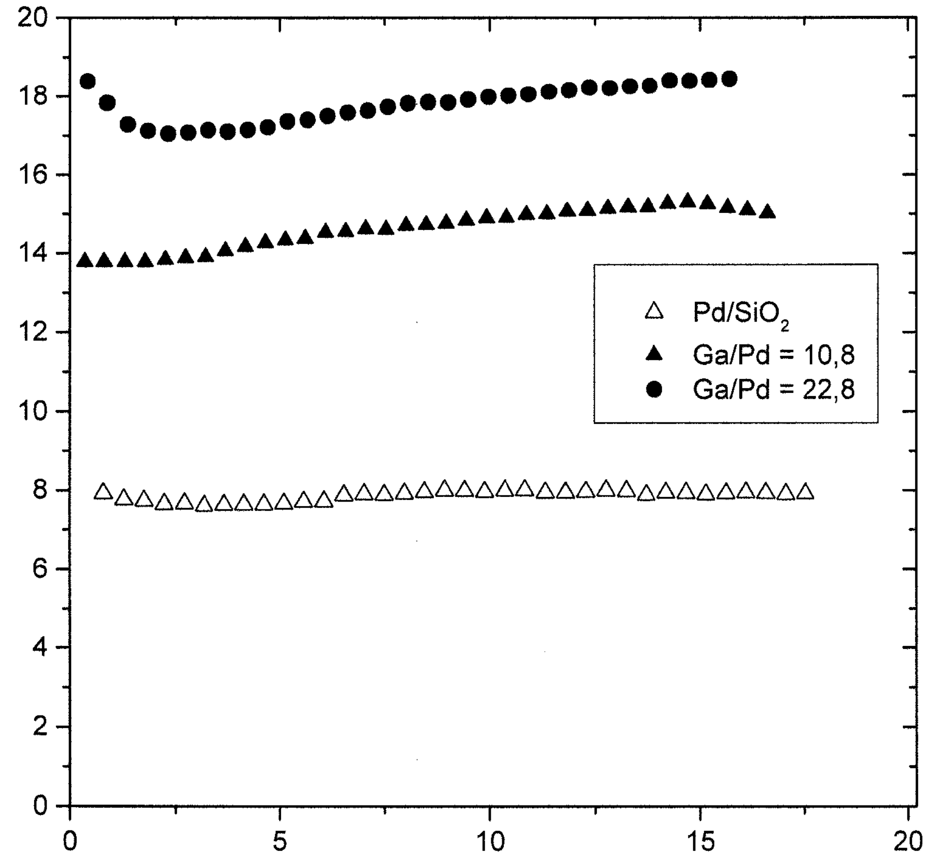
$Pd/SiO_2$
Ga/Pd = 10,8
Ga/Pd = 22,8

fig. 4

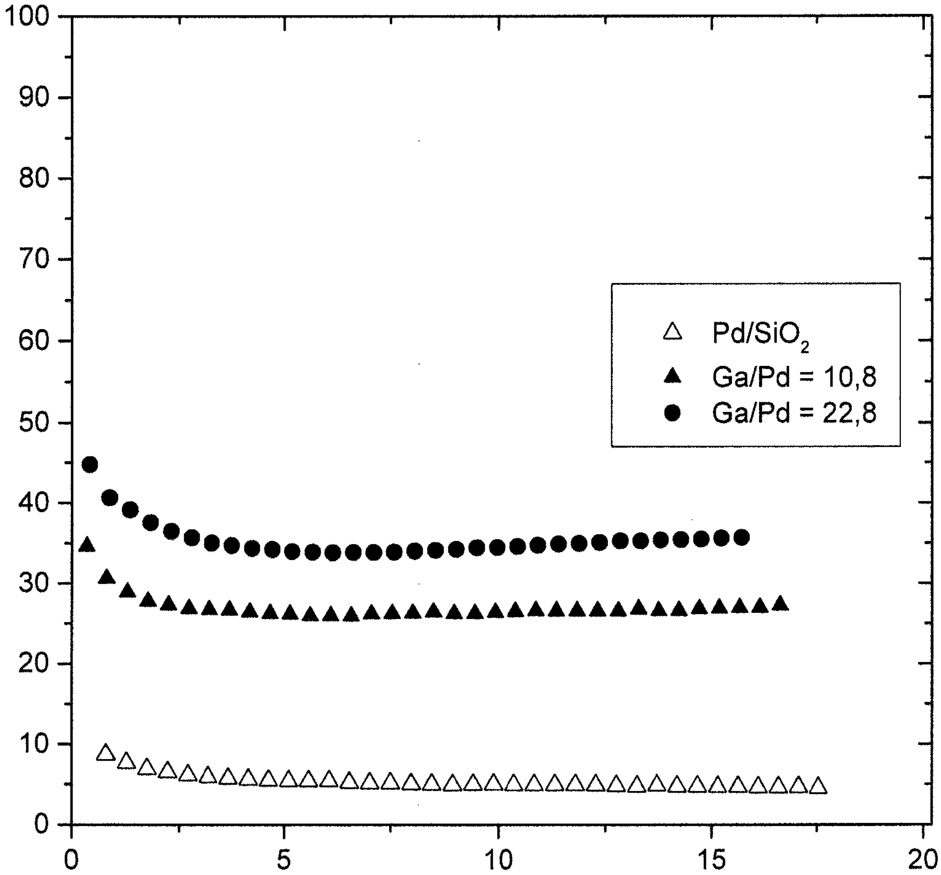


fig. 5

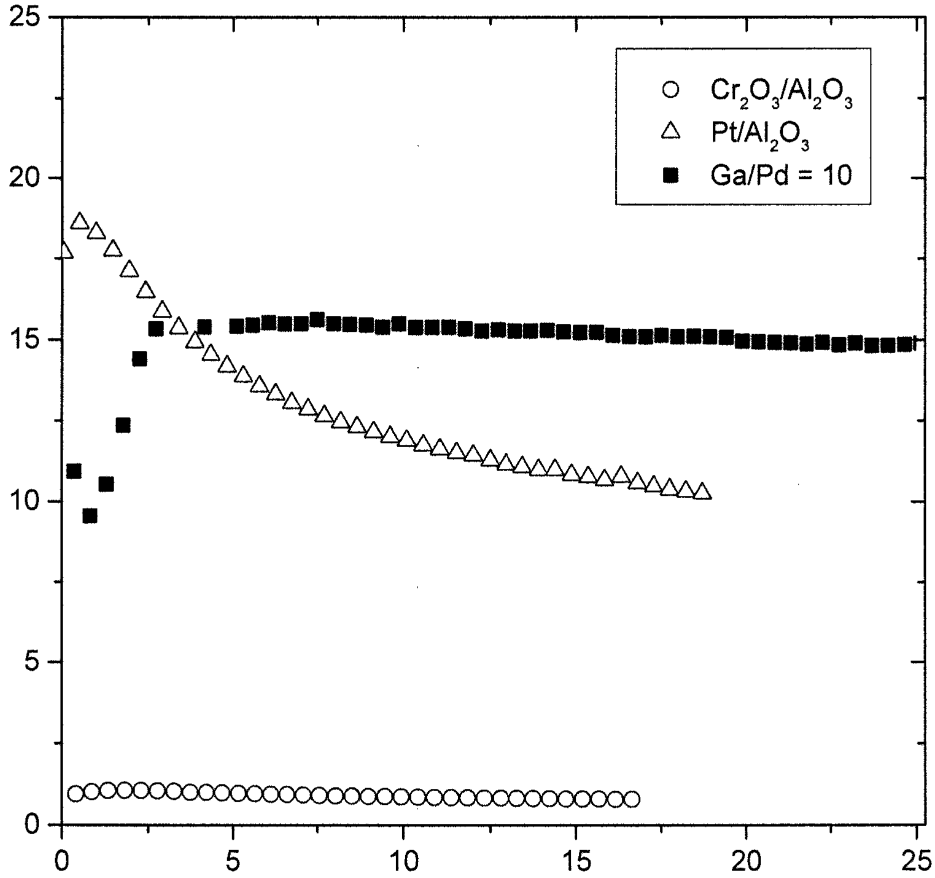


fig. 6

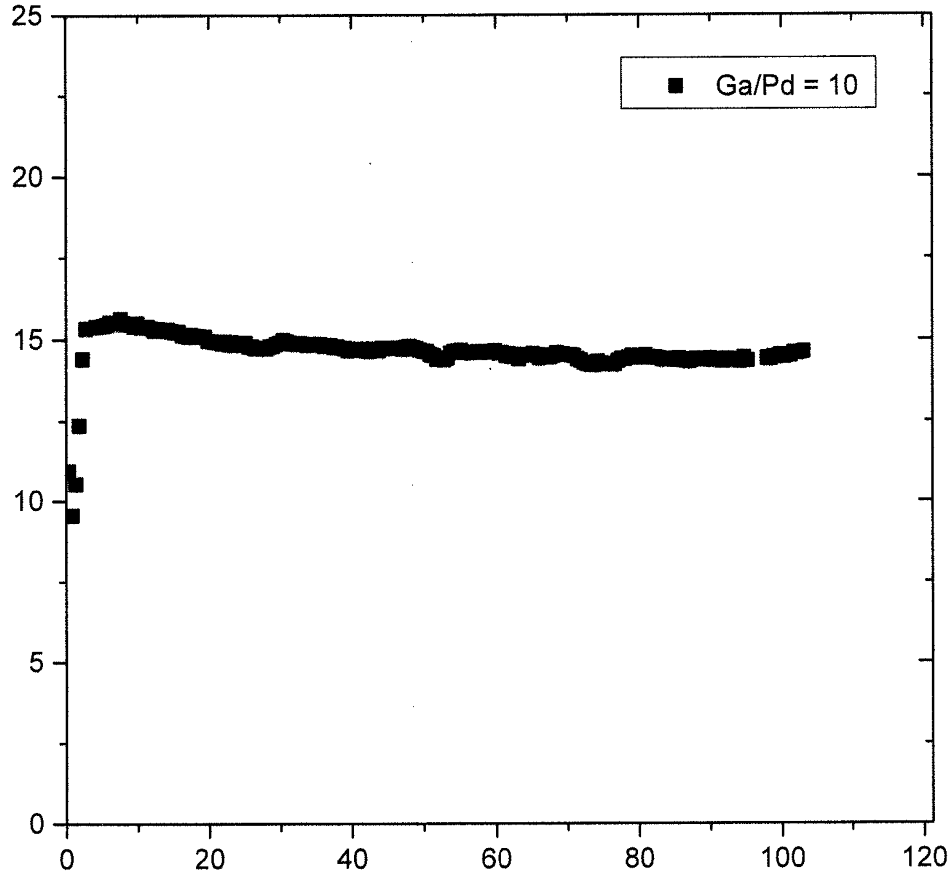
Ga/Pd = 10
0
5
10
15
20
25
0
20
40
60
80
100
120

fig. 7

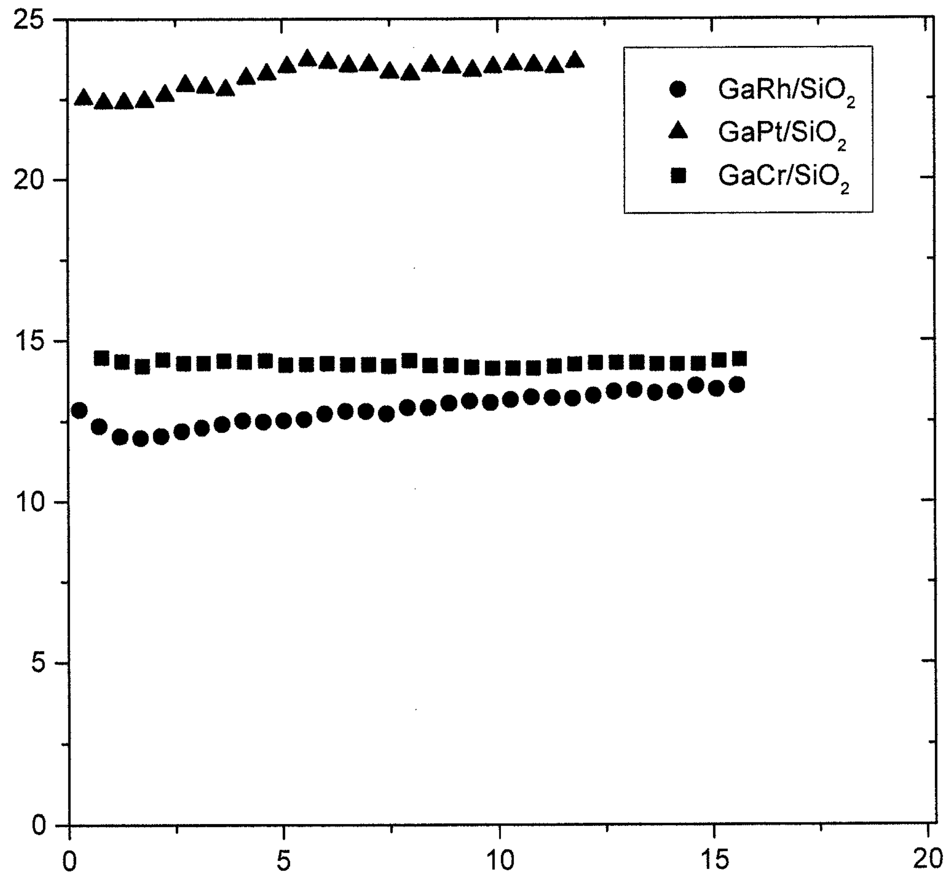
GaRh/SiO2
GaPt/SiO2
GaCr/SiO2
0
5
10
15
20
25
0
5
10
15
20

fig. 8

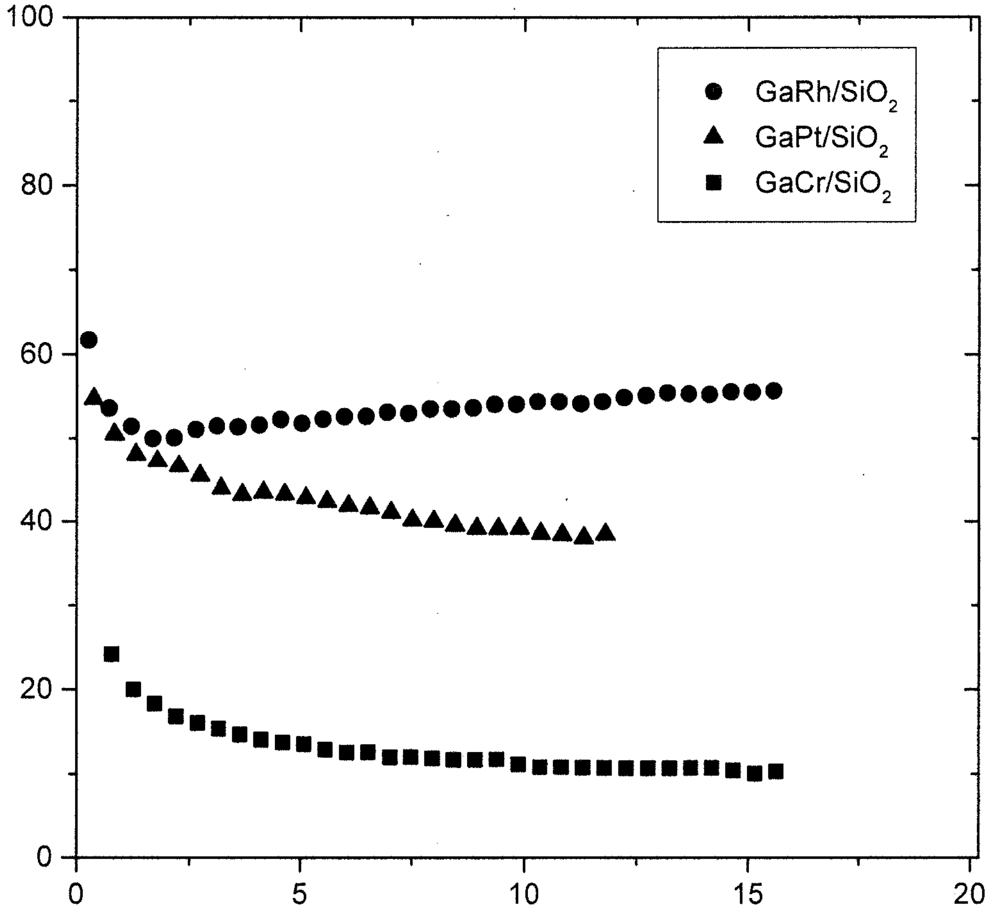
GaRh/$SiO_2$
GaPt/$SiO_2$
GaCr/$SiO_2$
0
20
40
60
80
100
0
5
10
15
20

fig. 9

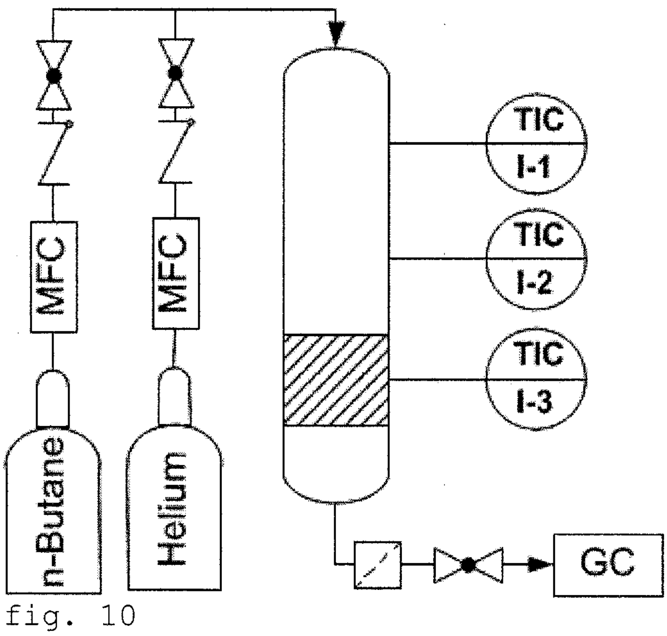
TIC
I-1
TIC
I-2
TIC
I-3
MFC
MFC
n-Butane
Helium
GC

fig. 10

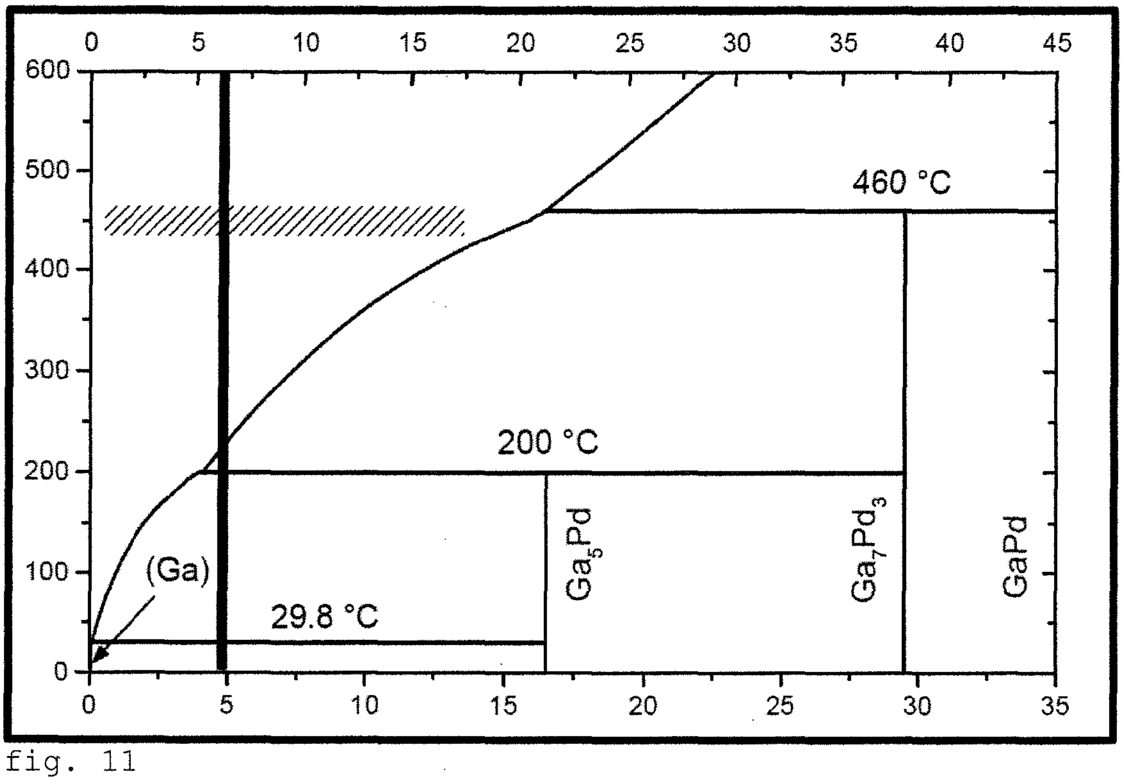
0
5
10
15
20
25
30
35
40
45
600
500
400
300
200
100
0
460 °C
200 °C
(Ga)
29.8 °C
$Ga_5Pd$
$Ga_7Pd_3$
GaPd
0
5
10
15
20
25
30
35

fig. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4822699 A, Wan
- US 20100236985 A, Luo
- US 20020198428 A
- US 4224192 A, Foster
- US 20070123418 A, Han
- US 20100303713 A, Zhang
- US 20100216630 A, Gajda
- EP 1533029 A, Benderly
- DE 1020090458004, Men
- US 4469812 A, Sorrentino
- US 1880711 A, Luczak

### Non-patent literature cited in the description

- **J. SATTLER et al.** *Chemical Reviews,* 2014, vol. 114, 10613-10653
- **S. VELDURTHI et al.** *Catalysis Today,* 2012, vol. 185, 88-93
- **S. BOCANEGRA et al.** *Catalysis Today,* 2009, vol. 143, 334-340
- **J. SATTLER et al.** *Physical Chemistry Chemical Physics,* 2013, 12095-12103
- **SHRIVER et al.** *Inorg. Synth.,* 1977, vol. 17, 42-45
- **S. WALTER et al.** *American Institute of Chemical Engineers Journal,* 2015, vol. 61, 893-897